# EUROPÄISCHE PATENTSCHRIFT

(11) **EP 1 337 288 B1**
(45) Veröffentlichungstag und Bekanntmachung des Hinweises auf die Patenterteilung: **12.03.2008**
(21) Anmeldenummer: 01995638.2
(22) Anmeldetag: 16.11.2001
(51) Int. Cl.: A61M 1/10

(54) **INTRAVASALE PUMPE**
INTRAVASCULAR PUMP
POMPE INTRAVASCULAIRE

(30) Priorität: 01.12.2000 DE 10059714
(43) Veröffentlichungstag der Anmeldung: 27.08.2003
(73) Patentinhaber: Abiomed Europe GmbH, 52074 Aachen (DE)
(72) Erfinder: SIESS, Thorsten, 52146 Würselen (DE); DÖPPER, Gebhard Dr., 74549 Wolpertshausen (DE)
(74) Vertreter: Selting, Günther
(86) Internationale Anmeldenummer: PCT/EP2001/013262
(87) Internationale Veröffentlichungsnummer: WO 2002/043791

(56) Entgegenhaltungen:
- EP-A- 0 629 412
- EP-A- 0 916 359
- EP-A- 1 034 808
- WO-A-00/43053
- WO-A-97/46270
- WO-A-99/44651
- DE-C- 19 821 307

## Beschreibung

Die Erfindung betrifft eine intravasale Pumpe mit einer Pumpeneinheit, die einen Antriebsteil und einen Pumpenteil aufweist, und einer von dem Pumpenteil abstehenden flexiblen Kanüle.

Eine intravasale Pumpe ist eine Pumpe, die durch das Blutgefäßsystem eines Patienten hindurch vorgeschoben werden kann, um beispielsweise in das Herz eingeführt zu werden. Eine solche intravasale Pumpe ist beschrieben in DE 198 21 307 C1. Diese Pumpe weist einen Antriebsteil mit einem Elektromotor und einen von dem Elektromotor angetriebenen Pumpenteil auf. An den Pumpenteil schließt sich eine langgestreckte Kanüle an, die derart platziert wird, dass sie durch mindestens eine Herzklappe hindurchgeht. Am distalen Ende der Kanüle befinden sich Öffnungen, durch die die Pumpe ansaugen oder ausstoßen kann. Die Pumpeneinheit ist mit einem Katheter verbunden, der unter anderem die Antriebsleitungen für den Elektromotor enthält. Der Außendurchmesser der Blutpumpe ist an keiner Stelle größer als 8 mm. Dadurch besteht die Möglichkeit, die Pumpe mit der Kanüle in dem Gefäßsystem eines Menschen vorzuschieben. Probleme bereitet aber das Einbringen der Pumpe in den Körper. Hierzu ist üblicherweise ein operativer Eingriff erforderlich. Es wäre erwünscht, eine Blutpumpe durch Punktion in den Körper einzubringen, beispielsweise durch die bei Kathetern übliche Seldinger-Technik. Hierzu haben die üblichen intravasalen Pumpen einen zu großen Durchmesser.' Eine Verkleinerung des Pumpenquerschnitts ist im Bereich der Kanüle nicht möglich, weil die Kanüle dann für den erforderlichen Durchfluss von 2 bis 2,5 Liter pro Minute zu eng würde. Infolge des hohen Strömungswiderstandes der Kanüle würde ein großer Teil der durch die Pumpe aufgebrachten Pumpleistung verloren gehen.

Eine intravasale Pumpe ist beschrieben in WO 97/46270. Diese Pumpe weist am Ende des Pumpenteils einen Ballonträger und einen darauf angeordneten aufblasbaren Ballon auf. Durch den Pumpenteil und den Ballonträger führt ein Kanal für einen Führungsdraht hindurch, der das Einbringen des Ballonkatheters in ein Blutgefäß erleichtert.

In DE 198 21 307 C1 ist eine intrakardiale Blutpumpe beschrieben, die aus einem Antriebsteil und einem Pumpenteil besteht, wobei am Pumpenteil eine flexible Kanüle vorgesehen ist. Die Kanüle trägt an ihrem distalen Ende eine Haltevorrichtung für ein Zugelement, an dem ein distaler Ballon befestigt ist. Über die Einführtechnik zum Einbringen der Pumpe in das Blutgefäßsystem enthält diese Schrift keine detaillierten Angaben.

EP 0 916 359 A1 beschreibt ebenfalls eine Pumpe mit Antriebsteil und Pumpenteil und einer von dem Pumpenteil abgehende Kanüle, die am distalen Ende ein starres Kopfstück aufweist. Die flexible Kanüle hat in Längsrichtung variierende Steifigkeit. Sie ist nicht kollabierbar. Über das Einführen der intravasalen Pumpe in das Gefäßsystem eines Patienten werden keine näheren Angaben gemacht.

In WO 99/44651 ist eine intravasal einführbare selbstentfaltbare Axialpumpe zur temporären Herzunterstützung beschrieben. Diese Pumpe weist ein selbstentfaltbares und radial komprimierbares flexibles Rohr in Kombination mit einem im Innern des Rohres befindlichen selbstentfaltbaren und radial komprimierbaren Rotor auf. Der Rotor ist mit einer Antriebswelle verbunden. Ein Deckschlauch schnürt das Rohr mit dem darin enthaltenen Rotor ein. Nach Platzierung der Pumpe im Herzen wird der Deckschlauch über einem Katheter zurückgezogen, wonach sich das flexible Rohr entfalten kann. Die radiale Komprimierbarkeit der Komponenten erlaubt eine Realisierung eines für eine perkutane Implantation in Seldinger-Technik vertretbar kleinen Punktionsdurchmessers.

Der Erfindung liegt die Aufgabe zugrunde, eine intravasale Pumpe mit flexibler Kanüle zu schaffen, die in der bei Kathetern üblichen Technik in den Körper eingebracht werden kann und dennoch das Pumpen mit hohen Fließraten ermöglicht.

Die Lösung dieser Aufgabe erfolgt erfindungsgemäß mit den im Patentanspruch 1 angegebenen Merkmalen. Hiernach besteht die Kanüle aus einem expandierbaren Schlauch, der einen Zustand mit einem relativ kleinen ersten Durchmesser und einen Zustand mit einem relativ großen zweiten Durchmesser annehmen kann. Die Kanüle weist ein starres Kopfstück auf, das beim Vorschieben der Pumpe durch einen Punktionskanal als Dilatator wirkt. Die Kanüle kann so eingeschnürt werden, dass sie mit dem kleinen ersten Durchmesser in den Körper und das Gefäßsystem eingeführt werden kann. Wenn sie sich im Gefäßsystem befindet, wird sie expandiert, so dass sie dann den für den Pumpenbetrieb erforderlichen größeren Durchmesser einnimmt. Die Kanüle ist formstabil, aber elastisch. Sie wird im gestreckten Zustand in den Körper eingebracht, nimmt aber dann, wenn die Pumpe bis in das Herz vorgeschoben ist, vorzugsweise eine gekrümmte Konfiguration an, die den Radien des Gefäßsystems entspricht. In Längsrichtung ist die Kanüle nicht wesentlich komprimierbar, so dass sie in einem Blutgefäß vorgeschoben werden kann, ohne wesentlich gestaucht zu werden.

Erfindungsgemäß ist vorgesehen, dass dass die Kanüle in einem schlauchförmigen Überzug in eingeschnürtem Zustand enthalten ist und bei Entfernen des Überzugs unter elastischer Aufweitung einen expandierten Zustand annimmt, und dass die Kanüle ein beim Vorschieben der Pumpe durch einen Punktionskanal als Dilatator dienendes starres Kopfstück aufweist.

Der Anmelderin ist es gelungen, die Abmessungen der rigiden Pumpeneinheit so zu reduzieren, dass der Durchmesser nicht größer ist als 4,0 mm. Die Pumpeneinheit kann daher nach Art eines Katheters durch Punktion eines Blutgefäßes, z.B. einer Vene, in den Körper eingeführt werden. Eine solche Blutpumpe hat ein Flügelrad, das mit hohen Drehzahlen von mindestens 30000 Umdrehungen pro Minute rotiert, typischerweise von 60000 Umdrehungen pro Minute. Die damit verbundene hohe Förderleistung erfordert eine Kanüle, deren Außendurchmesser größer ist als 4 mm. Die erfindungsgemäße Pumpe wird mit eingeschnürter Kanüle in den Körper eingebracht, wobei die Kanüle sich anschließend aufweitet, wenn sie sich in dem Blutgefäß befindet. Auf diese Weise reicht eine kleine Punktionsstelle aus. Ein Blutverlust und die mit Operationen stets verbundene Infektionsgefahr werden vermieden bzw. vermindert. Im expandierten Zustand ist der Durchmesser der Kanüle größer als derjenige des Antriebsteils.

Die Kanüle besteht aus einem in den expandierten Zustand vorgespannten elastischen Material, welches beim Einführen in den Körper mechanisch zusammengedrückt wird und im Innern des Blutgefäßes auseinandergeht.

Die Kanüle ist mit einem starren Kopfstück versehen. Das Kopfstück kann als Dilatator verwendet werden. Es ist mit einer Öffnung für den Durchtritt eines Führungsdrahtes versehen und erweitert sich kontinuierlich in proximaler Richtung. Eine derartige Kanüle kann auf einen perkutan in ein Blutgefäß hineinführenden Führungsdraht aufgeschoben werden und wirkt dann als Dilatator, der den den Führungsdraht enthaltenden Kanal aus Körpergewebe aufweitet und das Einschieben der gesamten Pumpe durch die aufgeweitete Öffnung ermöglicht.

Im folgenden werden unter Bezugnahme auf die Zeichnungen Ausführungsbeispiele der Erfindung näher erläutert.

Es zeigen:
- Fig. 1: eine Seitenansicht der intravasalen Pumpe im eingeschnürten Zustand,
- Fig. 2: eine Seitenansicht der Pumpe im expandierten Zustand,
- Fig. 3: eine Darstellung der Drahtstruktur der deformierbaren Kanüle,
- Fig. 4: einen Schnitt entlang der Linie IV-IV von Fig. 3, wobei die Drähte jedoch mit einem Mantel bzw. einer Haut umschlossen sind,
- Fig. 5: ein anderes, nicht zur Erfindung gehörendes Ausführungsbeispiel eines Gitters der Kanüle, wobei jedoch der äußere Mantel nicht dargestellt ist,
- Fig. 6: ein weiteres nicht zur Erfindung gehörendes Ausführungsbeispiel einer Tragstruktur der Kanüle im eingeschnürten Zustand,
- Fig. 7: die Tragstruktur nach Fig. 6 im expandierten Zustand,
- Fig. 8: das perkutane Punktieren eines Blutgefäßes und Einbringen eines Führungsdrahtes,
- Fig. 9: das Zurückziehen der für die Punktion benutzten Stahlkanüle
- Fig. 10: bei einem nicht zur Erfindung gehörenden Ausführungsbeispiel das Einbringen einer Schleuse in das Blutgefäß unter Verwendung eines Dilatators,
- Fig. 11: das Zurückziehen des Dilatators,
- Fig. 12: das Einschieben der Pumpe durch die Schleuse in das Gefäß, wobei die Kanüle der Pumpe sich im Innern des Blutgefäßes aufweitet,
- Fig. 13: eine erfindungsgemäße Ausführungsform, bei der die Kanüle mit einem aufreißbaren Überzug versehen ist, der sie im eingeschnürten Zustand hält und abgezogen werden kann,
- Fig. 14: das Zurückziehen des Überzuges von der Kanüle, und
- Fig. 15: das Vorschieben der Pumpe in dem Blutgefäß.

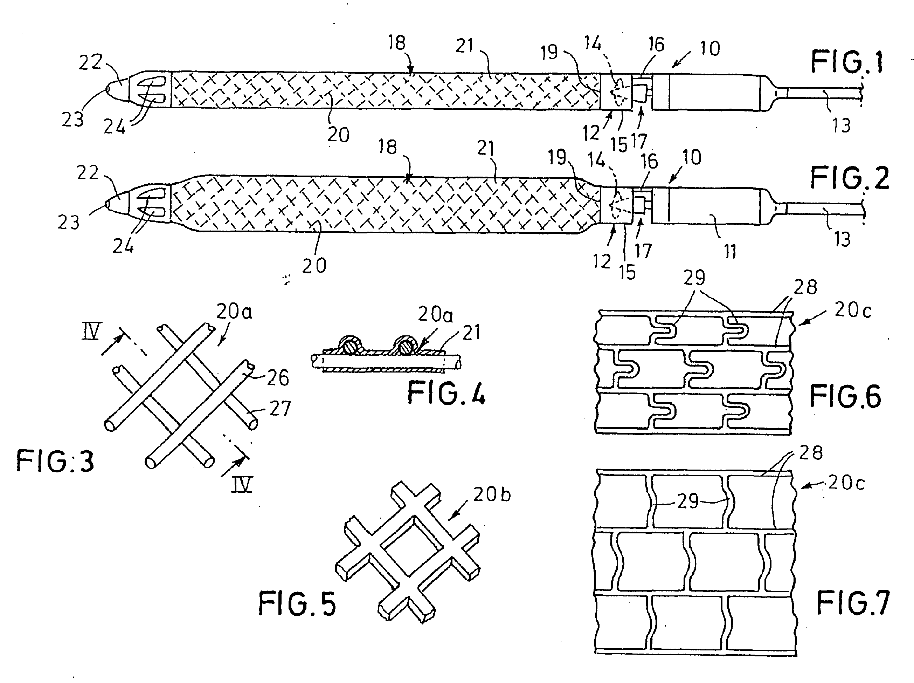

Die in den Fign. 1 und 2 dargestellte Pumpe weist eine Pumpeneinheit 10 aus einem Antriebsteil 11 und einem Pumpenteil 12 auf. Der Antriebsteil 11 ist zylindrisch ausgebildet mit einem Außendurchmesser von etwa 4 mm. Sein proximales Ende ist mit einem Katheter 13 verbunden, der ein durchgehendes Katheterlumen enthält und durch den auch die Drähte für die Versorgung des im Antriebsteil 11 enthaltenen Elektromotors verlaufen. Der Antriebsteil 11 treibt eine Welle, auf der ein im Pumpenteil 12 befindliches Flügelrad 14 sitzt. Das Flügelrad 14 rotiert im Innern eines Ringes 15, welcher das Pumpengehäuse bildet. Der Pumpenteil 12 ist in axialem Abstand von dem Antriebsteil 11 angeordnet und mit diesem durch längslaufende Stege 16 verbunden. Die Stege 16 überbrücken die Öffnung 17, die die Auslassöffnung oder die Einlassöffnung der Pumpe bildet, in Abhängigkeit von der Drehrichtung und Ausführungsform des Flügelrades 14. Wenn die Öffnung 17 die Einlassöffnung bildet, fördert das Flügelrad 14 das Blut in axialer Richtung in die Kanüle 18, die sich an die axiale Öffnung 19, welche die Auslassöffnung bildet, anschließt. Der Blutfluss kann auch umgekehrt stattfinden.

Die Kanüle 18 bildet einen Pumpenschlauch mit einer Länge von etwa 50 bis 60 mm. Sie ist formstabil, jedoch elastisch. Die Kanüle 18 enthält eine Tragstruktur 20 und einen die Tragstruktur bedeckenden geschlossenen Mantel 21. Am distalen Ende der Kanüle 18 befindet sich ein starres Kopfstück 22, das sich vom distalen Ende nach proximal hin erweitert und eine Öffnung 23 für den Durchtritt eines Führungsdrahtes aufweist. An dem Kopfstück 22 befinden sich weitere Öffnungen 24 für den Durchtritt des zu pumpenden Blutes.

In dem in Fig. 1 dargestellten Zustand hat die Kanüle 18 einen geringen Außendurchmesser, der ungefähr dem Außendurchmesser der Pumpeneinheit 10 entspricht, im vorliegenden Fall 4 mm. In diesem Zustand ist die Kanüle 18 flexibel, so dass sie zum Einführen in das Gefäßsystem gebogen werden kann.

In Fig. 2 ist die Pumpe 10 mit expandierter Kanüle 18 dargestellt. Hierbei beträgt der Außendurchmesser der Kanüle 18 etwa 5,4 mm. Die Kanülenlänge ist gegenüber Fig. 1 unverändert. Dieser Zustand ist der Betriebszustand der Kanüle, den diese bei laufender Pumpeneinheit 10 einnimmt.

In den Fign. 3 und 4 ist ein Ausführungsbeispiel der Tragstruktur 20a der Kanüle 18 dargestellt. Die Tragstruktur 20a besteht aus sich kreuzenden elastischen Drähten 26 und 27, die eine Parallelogrammstruktur bilden. Die Drähte 26,27 sind schraubenförmig gewickelt und durch den aus Kunststoff bestehenden Mantel 21 miteinander verbunden. An den Kreuzungspunkten haben sie eine solche Orientierung, dass die Kanüle 18 bestrebt ist, den expandierten Zustand einzunehmen. Andererseits kann durch Zusammendrücken die Kanüle in den eingeschnürten Zustand gebracht werden.

Fig. 5 zeigt eine ähnliche Tragstruktur 20b aus einem Gitter, dessen Stäbe ebenfalls diagonal verlaufen und eine Parallelogrammstruktur bilden. Die Gitterstäbe liegen in derselben Ebene. Das Gitter kann beispielsweise durch Laserschneiden aus einem Rohr hergestellt werden. Es besteht aus einer Metalllegierung mit Formgedächtnis, beispielsweise aus Nitinol. Bei Raumtemperatur ist das Gitter im zusammengeschnürten Zustand plastisch "eingefroren" und kann durch Erwärmen auf Körpertemperatur superelastisch expandiert werden.

Die Fign. 6 und 7 zeigen eine weitere Ausführungsform einer Tragstruktur 20c mit Formgedächtnis. Diese Tragstruktur enthält längslaufende gerade Stege 28, die durch querlaufende Schleifenstege 29 verbunden sind. Die Tragstruktur bildet ein Rohr. Die Schleifenstege 29 haben ein Formgedächtnis. Sie sind in dem in Fig. 6 dargestellten stark gebogenen Zustand eingefroren und können durch Wärme auf den in Fig. 7 dargestellten gestreckten Zustand aufgeweitet werden. Auch hier ist ein aus Kunststoff bestehender Mantel vorgesehen, der mit der Tragstruktur 20c vereinigt ist.

Statt einem Material mit Formgedächtnis kann nach der Erfindung ein elastischer Federstahl verwendet werden, der bei entsprechender Ausformung nur im elastischen Bereich deformiert wird und somit nach erfolgter Zusammendrückung in den expandierten Zustand übergeht.

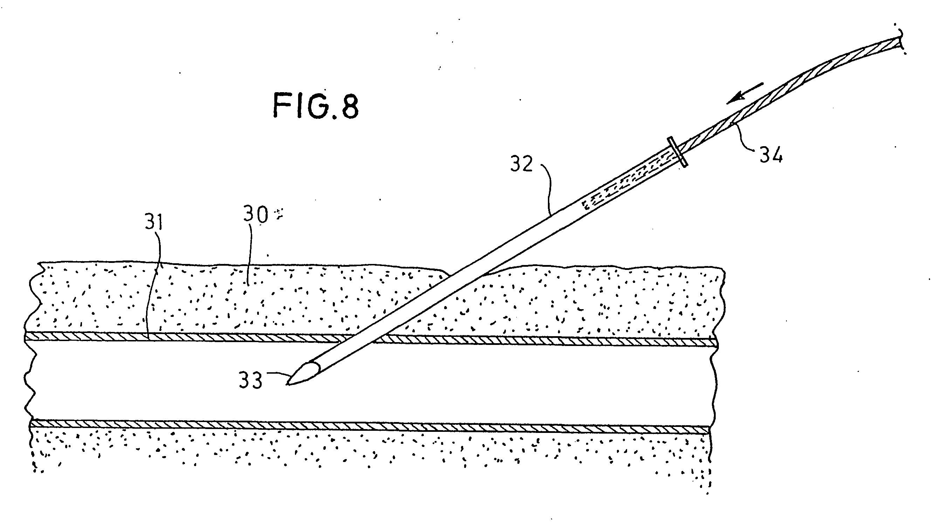

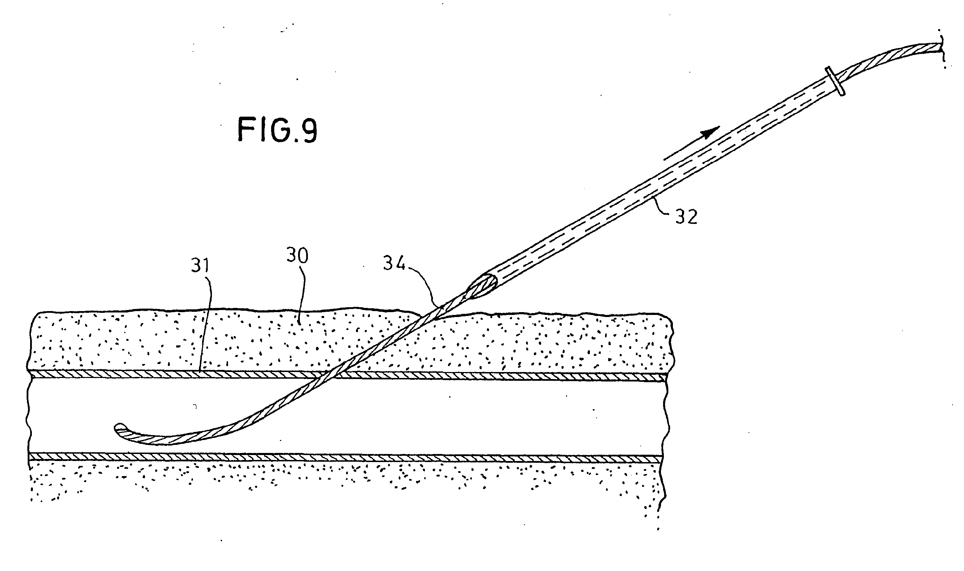

Die Fign. 8 bis 12 zeigen eine erste Technik des Einführens der Pumpe in das Blutgefäßsystem. Zunächst wird gemäß Fig. 8 durch die Haut 30 hindurch ein Blutgefäß 31 mit einer Stahlkanüle 32 mit schneidender Spitze 33 punktiert. Durch die Stahlkanüle 32 hindurch wird ein Führungsdraht 34 eingeführt und dann gemäß Fig. 9 in dem Blutgefäß 31 vorgeschoben. Danach wird die Stahlkanüle zurückgezogen.

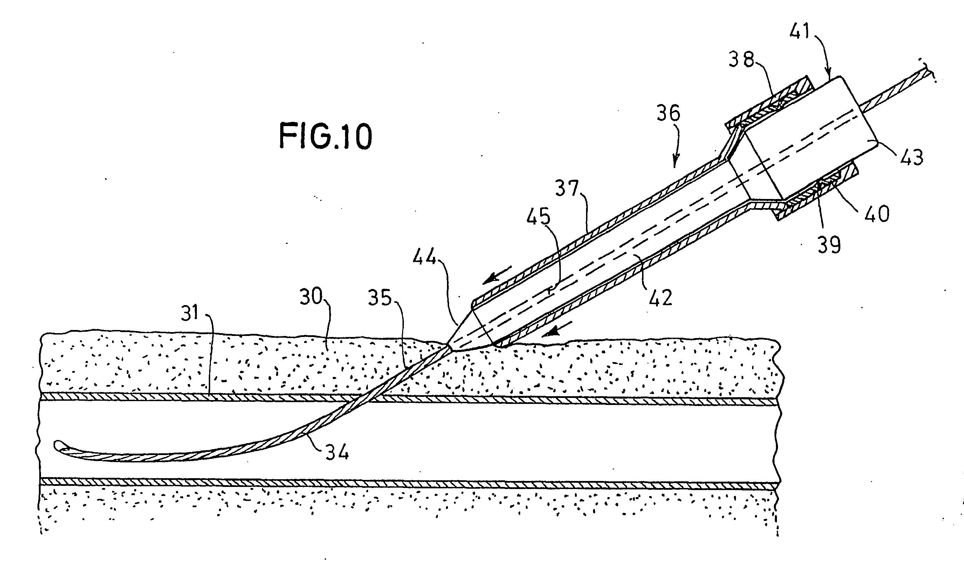

Gemäß Fig. 10 wird in den Punktionskanal 35, durch den der Führungsdraht 34 hindurchgeht, eine Schleuse 36 eingeführt. Die Schleuse 36 besteht aus einem relativ steifen Rohr 37, dessen Innendurchmesser etwas größer ist als 4 mm, und einem am proximalen Ende des Rohres 37 angeordneten hämostatischen Ventil 38.

Das hämostatische Ventil 38 enthält ein elastomeres Ringteil 39, welches durch Verdrehen einer Schraubkappe 40 axial zusammengequetscht wird und gleichzeitig radial nach innen ausweicht.

In der Schleuse 36 sitzt ein Dilatator 41, der einen das Rohr 37 ausfüllenden Schaft 42 und am rückwärtigen Ende einen in dem Ventil 38 sitzenden Kopf 43 aufweist. Das Ringteil 39 des Ventils wird beim Spannen der Schraubkappe 40 gegen den Kopf 43 gedrückt, so dass aus der Schleuse kein Blut austreten kann. Der Dilatator 41 weist eine aus dem Rohr 37 vorstehende konische Spitze 44 auf. Durch die Länge des Dilatators hindurch verläuft ein Kanal 45, durch den der Führungsdraht 34 geschoben werden kann. Der Dilatator 41 dient zum Aufweiten des Punktionskanals 35 und zum Einbringen der Spitze der Schleuse 36 in das Blutgefäß 31.

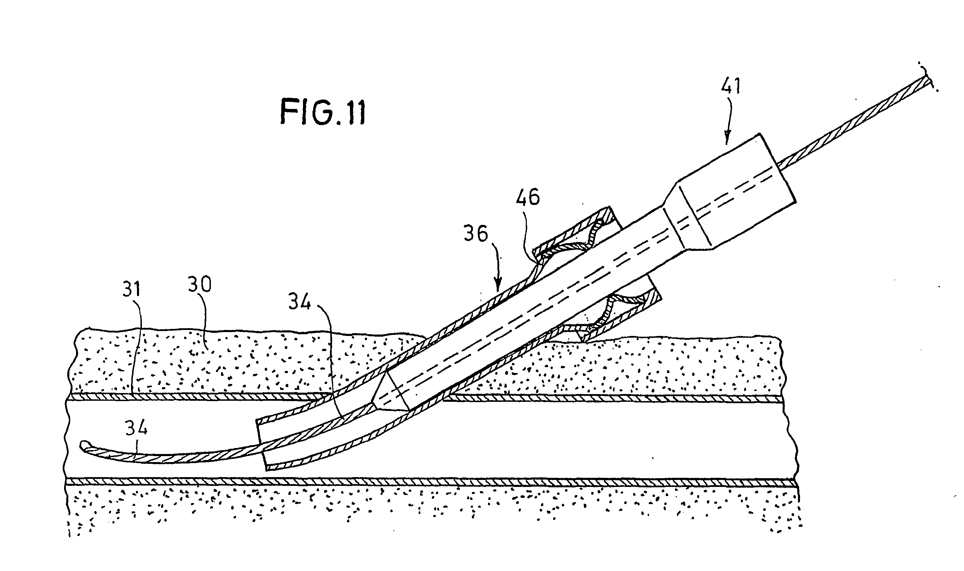

Fig. 11 zeigt das Zurückziehen des Dilatators 41 aus der Schleuse 36, so dass diese nun für das Einführen der Pumpe 10 zur Verfügung steht.

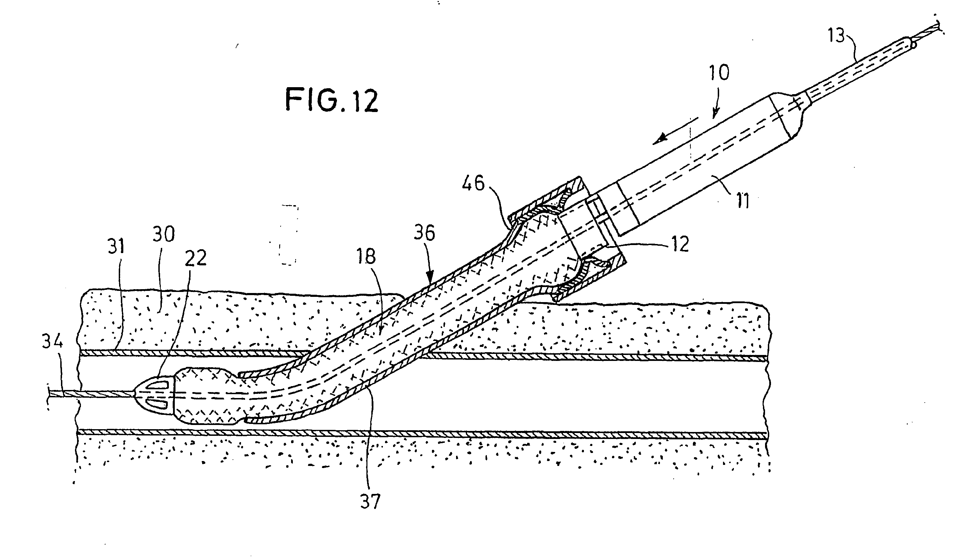

Gemäß Fig. 12 wird die Pumpe 10 in der Weise in die Schleuse 36 eingeführt, dass zunächst der Kopf 22 in das proximale Ende der Schleuse eingeschoben wird. Der Kopf 22 hat denselben maximalen Außendurchmesser wie die Pumpeneinheit 10. Dann folgt der übrige Teil der Kanüle 18, welcher durch den Verjüngungsbereich 46 am rückwärtigen Schleusenende radial zusammengedrückt und im zusammengedrückten Zustand durch das Rohr 37 geschoben wird. In dem Blutgefäß tritt die Kanüle 18 aus der Schleuse 36 aus und erweitert sich dann infolge ihrer elastischen Vorspannung wieder. Über dem Führungsdraht 34 wird auf diese Weise die gesamte Pumpe mit Kanüle 18 und Pumpeneinheit 10 in das Blutgefäß eingeschoben. Danach wird die Schleuse 36 und der Führungsdraht 34 entfernt.

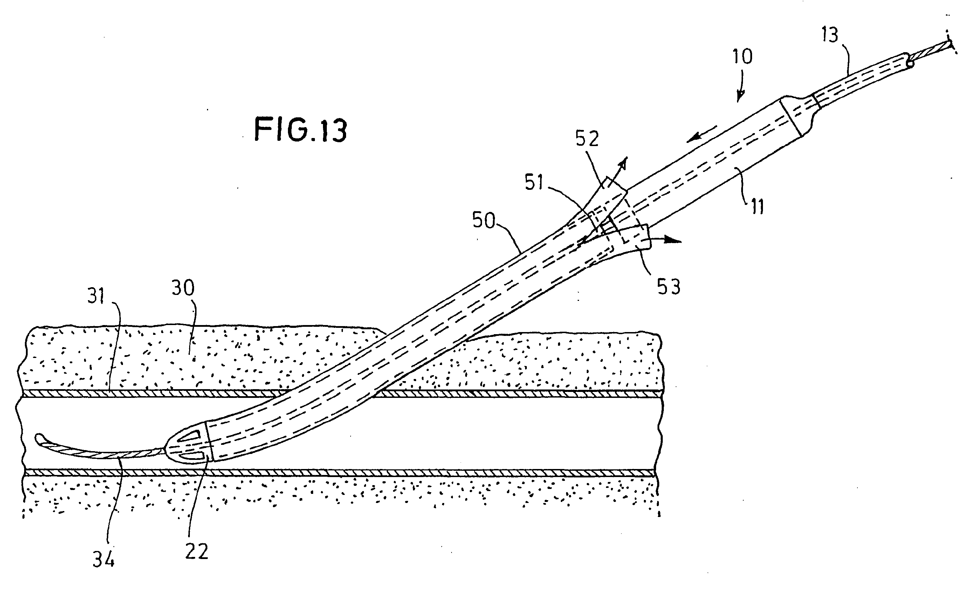

Die Fign. 13 bis 15 zeigen das Einbringen der erfindungsgemäßen Pumpe 10 in das Blutgefäß 31. Die Kanüle ist hierbei mit einem schlauchförmigen Überzug 50 im eingeschnürten Zustand gehalten. Der Überzug 50 besteht aus einem dünnwandigen Schlauch, der am rückwärtigen Ende zwei längslaufende Anrisse 51 aufweist, welche das Auftrennen des Schlauchs in zwei separate Bahnen 52,53 ermöglichen. Der Überzug 50 besteht aus einer Folie, deren Molekularstruktur in Längsrichtung des Schlauchs gerichtet ist, so dass sich beim Auseinanderziehen der Bahnen 52,53 der jeweilige Riss in Längsrichtung fortsetzt.

Beim Einführen der Pumpe über dem Führungsdraht 34 dient der Kopf 22 als Dilatator, der den Punktionskanal beim Vorschieben der Pumpe 10 aufweitet. Hierzu ist weder eine Schleuse, noch ein separater Dilatator erforderlich. Der Überzug 50 wird, wenn der in Fig. 13 dargestellte Zustand erreicht ist, in proximaler Richtung abgezogen, während die Pumpe 10 weiter über dem Führungsdraht 34 vorgeschoben wird. Beim Zurückziehen des Überzugs 50 wird dieser gesplittet. Auf diese Weise wird die gesamte Pumpe unter gleichzeitigem Zurückziehen des Überzugs 50 in das Blutgefäß eingebracht. Der Außendurchmesser des Überzugs 50 beträgt im Bereich der Kanüle etwa 3,0 mm, so dass sich die Anrisse 51 beim Zurückziehen des Schlauchs über den ca. 1 mm größeren Pumpenteil automatisch fortpflanzen.

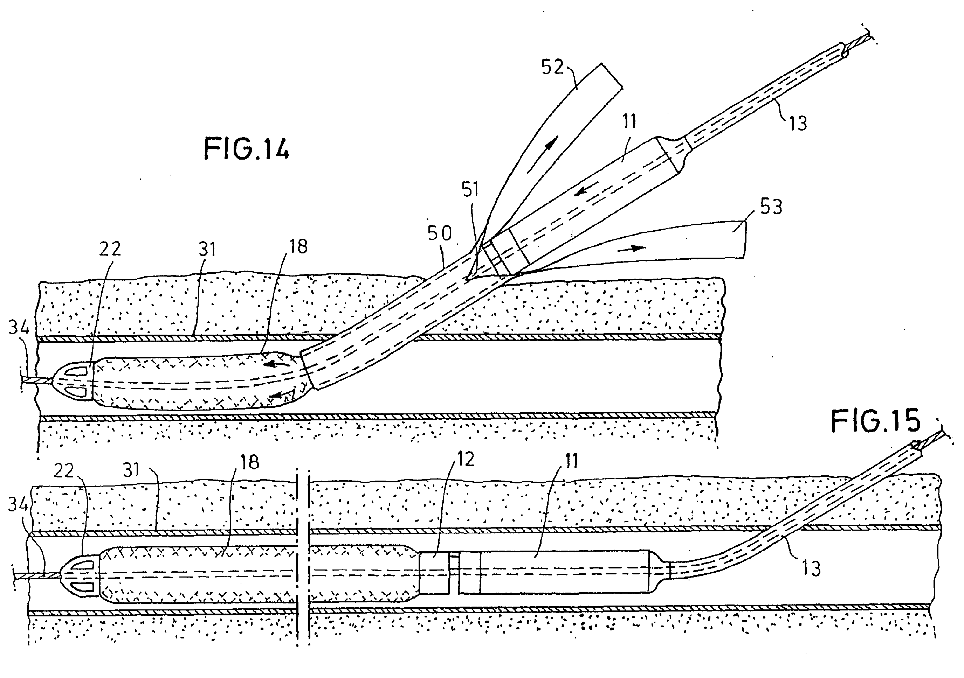

Das Fördervolumen der Pumpe in dem in Fig. 15 dargestellten Zustand beträgt typischerweise 2,0 bis 2,5 Liter pro Minute.

Wenn die Kanüle aus einem elastisch aufweitbaren Material besteht, also nicht einem Material mit Formgedächtnis, enthält sie vorzugsweise einen Federstahldraht, der schraubenförmig gewickelt sein kann. Durch Verschweißen einzelner Drähte oder durch einen unterschiedlich dicken Kunststoffmantel kann die Steifigkeit der Kanüle über die Kanülenlänge variieren.

## Patentansprüche

1. Intravasale Pumpe mit einer Pumpeneinheit (10) aus einem Antriebsteil (11) und einem damit verbundenen Pumpenteil (12) und mit einer von dem Pumpenteil (12) abgehenden flexiblen Kanüle (18) aus einem expandierbaren Schlauch, der einen Zustand mit einem relativ kleinen ersten Durchmesser und einen Zustand mit einem relativ großen zweiten Durchmesser annehmen kann, wobei die Kanüle (18) sich an eine axiale Öffnung (19) des Pumpenteils (12) anschließt und von diesem in distaler Richtung absteht,
und wobei die Kanüle (18) in einem schlauchförmigen Überzug (50) in eingeschnürtem Zustand enthalten ist und bei Entfernen des Überzugs (50) unter elastischer Aufweitung einen expandierten Zustand annimmt, und wobei die Kanüle (18) ein starres Kopfstück (22) aufweist, das beim Vorschieben der Pumpe durch einen Punktionskanal als Dilatator dient.

2. Pumpe nach Anspruch 1, **dadurch gekennzeichnet, dass** das Kopfstück (22) eine Öffnung (23) für den Durchtritt eines Führungsdrahtes (34) aufweist.

3. Pumpe nach Anspruch 1 oder 2, **dadurch gekennzeichnet, dass** die Kanüle radial federelastisch ist.

4. Pumpe nach einem der Ansprüche 1-3, **dadurch gekennzeichnet, dass** der Überzug (50) längslaufende Anrisse (51) aufweist, die ein Aufreißen in zwei separate Bahnen (52,53) ermöglichen.

5. Pumpe nach einem der Ansprüche 1-4, **dadurch gekennzeichnet, dass** die Steifigkeit der Kanüle (18) von der Kanülenspitze aus nach hinten zunimmt.

## Claims

1. An intravascular pump comprising a pump unit (10) including a drive portion (11) and a pump portion (12) connected therewith, and comprising a flexible cannula (18) extending from the pump portion (12) and consisting of an expandable hose adapted to assume a state with a relatively small first diameter and a state with a relatively large second diameter, the cannula (18) connecting to an axial opening (19) of the pump portion (12) and extending from the pump portion (12) in a distal direction, and the cannula (18) being contained in a tubular envelope (50) in a constricted state and, upon removal of the envelope (50), assuming an expanded state while dilating elastically, and the cannula (18) comprising a rigid head piece (22) serving as a dilator during the advancement of the pump through a puncturing channel.

2. The pump according to claim 1, **characterized in that** the head piece (22) comprises an opening (23) for the passage of a guide wire (34).

3. The pump according to claim 1 or 2, **characterized in that** the cannula is radially spring-elastic.

4. The pump according to any one of claims 1 - 3, **characterized in that** the envelope (50) comprises longitudinally extending fissures (51) permitting a tear into two separate sheets (52,53).

5. The pump according to any one of claims 1 - 4, **characterized in that** the rigidity of the cannula (18) increases from the cannula tip to the rear.

## Revendications

1. Pompe intravasculaire comprenant une unité de pompage (10), constituée d'une partie motrice (11) et d'une partie de pompage (12) reliée à cette dernière, et une canule (18) flexible constituée d'un boyau expansible, qui part de la partie de pompage (12) et qui peut adopter un état avec un premier diamètre, relativement petit, et un état avec un deuxième diamètre, relativement grand, la canule (18) se raccordant à une ouverture axiale (19) de la partie de pompage (12) et s'éloignant de celle-ci en direction distale,
et dans laquelle la canule (18) est, dans l'état contracté, contenue dans une enveloppe (50) en forme de boyau et, lorsque l'enveloppe (50) est enlevée, elle adopte, sous élargissement élastique, un état expansé, et dans laquelle la canule (18) présente une pièce frontale (22) rigide, qui, lors de l'avancement de la pompe à travers un canal de ponction, fait office de dilatateur.

2. Pompe selon la revendication 1, **caractérisée en ce que** la pièce frontale (22) présente un orifice (23) pour le passage d'un fil directeur (34).

3. Pompe selon la revendication 1 ou 2, **caractérisée en ce que** la canule possède une élasticité radiale similaire à celle d'un ressort.

4. Pompe selon l'une des revendications 1 à 3, **caractérisée en ce que** l'enveloppe (50) présente des amorces de rupture (51) longitudinales, qui permettent un déchirement en deux bandes séparées (52, 53).

5. Pompe selon l'une des revendications 1 à 4, **caractérisée en ce que** la rigidité de la canule (18) croît de la pointe de la canule vers l'arrière.
